# EUROPEAN PATENT APPLICATION

(11) **EP 3 263 141 A1**
(43) Date of publication of application: **03.01.2018**
(21) Application number: 16755628.1
(22) Date of filing: 25.02.2016
(51) Int. Cl.: A61L 27/00

(54) **MEDICAL MATERIAL AND ANTI-ADHESION MATERIAL**

(30) Priority: 27.02.2015 JP 2015038007
(71) Applicant: Dainichiseika Color & Chemicals Mfg. Co., Ltd., Chuo-ku Tokyo 103-8383 (JP)
(72) Inventor: ISONO, Yasuyuki, Tokyo 103-8383 (JP); NOISHIKI, Yasuharu, Yokohama-shi Kanagawa 236-0005 (JP)
(74) Representative: Schiener, Jens
(86) International application number: PCT/JP2016/055654
(87) International publication number: WO 2016/136884

(57) **Abstract**

There is provided a medical material that retains the properties which are inherent in a polyanionic polysaccharide being a raw material, that has a high level of safety because there is no need to use a chemical crosslinking agent during production, and that has a moderate strength with which a suture and the like can be conducted. The medical material is provided with a reinforcement material and a film section penetrating at least a portion of the reinforcement material to be integrated with the reinforcement material. In the medical material, the film section is formed by water-insolubilizing a film precursor section made of a raw material containing a water-soluble salt of a first polyanionic polysaccharide with a treatment liquid containing a first acid anhydride, and the reinforcement material is nonwoven fabric or woven fabric containing a biodegradable/absorbent polymer material, or a sponge-like carrier or a felt-like carrier formed by water-insolubilizing a raw material-shaped body made of a raw material containing a water-soluble salt of a second polyanionic polysaccharide with a treatment liquid containing a second acid anhydride.

## Description

### Technical Field

The present invention relates to a medical material and an anti-adhesion material.

### Background Art

It is known that polyanionic polysaccharides such as hyaluronic acid and alginic acid exhibit moderate viscosity, adhesiveness, moisture retention, and biocompatibility. Therefore, these polyanionic polysaccharides and salts thereof are widely used as a raw material for medical materials, food materials, cosmetic materials, and the like.

Hyaluronic acid among others is excellent in its characteristic physical properties such as water retention and has a high level of safety and biocompatibility, so that it is used in various applications such as foods, cosmetics, and pharmaceutical products. For example, in the medical field, hyaluronic acid is used in raw materials for joint lubricants and anti-adhesion materials. However, sodium hyaluronate to be a raw material has a high water-solubility and therefore needs to be subjected to certain insolubilization treatment depending on the application.

Various studies on the method for water-insolubilizing sodium hyaluronate through crosslinking reaction making use of a carboxy group have been made so far. For example, Patent Literature 1 describes a method for producing a water-insoluble derivative of a polyanionic polysaccharide such as hyaluronic acid and carboxymethyl cellulose through crosslinking reaction using a carbodiimide.

In addition, Patent Literatures 2 and 3 describe a method for water-insolubilizing a polyanionic polysaccharide such as hyaluronic acid and carboxyalkyl cellulose by forming ionic bonds using a polyvalent cation. Further, Patent Literature 4 describes a method for obtaining a water-insolubilized film by subjecting carboxymethyl cellulose to ion exchange using a metal salt.

Patent Literature 5 describes a method for water-insolubilizing sodium hyaluronate by cooling a sodium hyaluronate aqueous solution to -20°C under an acidic condition to form intramolecular crosslinks. In addition, Patent Literature 6 describes acetylation through reaction of hyaluronic acid in a powder form with acetic anhydride in the presence of concentrated sulfuric acid. Further, Patent Literature 7 describes a method for producing hyaluronic acid gel using an acidic liquid containing an alcohol.

### Citation List

### Patent Literature

Patent Literature 1: National Publication of International Patent Application No. 2003-518167
Patent Literature 2: Japanese Patent Application Laid-Open No. 5-124968
Patent Literature 3: Japanese Patent Application Laid-Open No. 2008-13510
Patent Literature 4: Japanese Patent Application Laid-Open No. 6-128395
Patent Literature 5: Japanese Patent Application Laid-Open No. 2003-252905
Patent Literature 6: Japanese Patent Application Laid-Open No. 8-53501
Patent Literature 7: Japanese Patent Application Laid-Open No. 5-58881

### Summary of Invention

### Technical Problem

However, a crosslinking agent is used in the method described in Patent Literature 1, and therefore it is often difficult to apply the method for applications where the safety should be taken into consideration because products are applied to human bodies, such as pharmaceutical products. In addition, the extent of water-insolubility for the obtained films and the like are not described at all in Patent Literatures 2 to 4.

Further, in the method described in Patent Literature 5, the pH of the sodium hyaluronate aqueous solution needs to be adjusted at about 1.2, and the viscosity increases outstandingly, so that handling at the time of shaping and the like is difficult. In addition, freeze drying is conducted for long hours, so that there has also been a problem in terms of cost of electric power required for cooling. Further, when the sodium hyaluronate aqueous solution is stored under an acidic condition, the viscosity increases rapidly to make the shaping difficult, so that the application may be limited. It is to be noted that in Patent Literature 5, the intramolecular crosslinked structure is identified, but the extent of insolubilization is not referred to.

In addition, the extent of water-insolubility for the obtained acetylated product of hyaluronic acid is not described at all in Patent Literature 6. Further, the hyaluronic acid gel obtained by the method described in Patent Literature 7 contains a large amount of water and therefore is hard even to lift. Thus, it is difficult to insolubilize the shaped body while keeping the shape of the shaped body.

The present invention has been completed in consideration of the problems of the conventional techniques, and the subject matter of the present invention is to provide a medical material and an anti-adhesion material: that retain the properties which are inherent in a polyanionic polysaccharide being a raw material, that have a high level of safety because there is no need to use a chemical crosslinking agent during production; and that have a moderate strength with which a suture and the like can be conducted.

### Solution to Problem

That is, according to the present invention, a medical material described below is provided.
[1] A medical material provided with: a reinforcement material; and a film section penetrating at least a portion of the reinforcement material to be integrated with the reinforcement material, wherein: the film section is formed by water-insolubilizing a film precursor section made of a raw material containing a water-soluble salt of a first polyanionic polysaccharide with a treatment liquid containing a first acid anhydride; and the reinforcement material is: nonwoven fabric or woven fabric containing a biodegradable/absorbent polymer material; or a sponge-like carrier or a felt-like carrier formed by water-insolubilizing a raw material-shaped body made of a raw material containing a water-soluble salt of a second polyanionic polysaccharide with a treatment liquid containing a second acid anhydride.
[2] The medical material according to [1], wherein a mass per unit area of the film section is 5 to 100 g/m².
[3] The medical material according to [1] or [2], having a threading tension of 0.1 to 20 N/cm.
[4] The medical material according to any one of [1] to [3], wherein the first polyanionic polysaccharide and the second polyanionic polysaccharide are each at least one selected from the group consisting of hyaluronic acid, carboxymethyl cellulose, and alginic acid.
[5] The medical material according to any one of [1] to [4], wherein the first acid anhydride and the second acid anhydride are each at least any one of acetic anhydride and propionic anhydride.
[6] The medical material according to any one of [1] to [5], wherein the polymer material is at least one selected from the group consisting of polylactic acid, lactic acid-caprolactone copolymers, and polyglycolic acid.
   In addition, according to the present invention, an anti-adhesion material described below is provided.
[7] An anti-adhesion material containing a polyhydric alcohol or a polyhydric alcohol aqueous solution retained in the medical material according to any one of [1] to [6].

### Advantageous Effects of Invention

The medical material and the anti-adhesion material according to the present invention each retain the properties which are inherent in a polyanionic polysaccharide being a raw material, each have a high level of safety because there is no need to use a chemical crosslinking agent during production, and each have a moderate strength with which a suture and the like can be conducted.

### Description of Embodiments

Hereinafter, embodiments according to the present invention will be described; however, the present invention is not limited to the embodiments below.

### (Medical Material)

A medical material according to the present invention is provided with a reinforcement material and a film section penetrating at least a portion of the reinforcement material to be integrated with the reinforcement material. The film section is formed by insolubilizing a film precursor section with a predetermined treatment liquid. The film precursor section is formed with a raw material containing a water-soluble salt of a first polyanionic polysaccharide. The first polyanionic polysaccharide is a polysaccharide having one or more negatively charged anionic groups such as a carboxy group and a sulfonic acid group in the molecular structure thereof. In addition, the water-soluble salt of the first polyanionic polysaccharide is a salt formed from at least a part of the anionic groups in the first polyanionic polysaccharide. It is to be noted that the anionic group in the first polyanionic polysaccharide may be the one that is introduced in a molecule of a polysaccharide.

Specific examples of the first polyanionic polysaccharide include carboxyalkyl cellulose such as carboxymethyl cellulose and carboxyethyl cellulose, carboxymethyl starch, carboxymethylamylose, chondroitin sulfate (including chondroitin-4-sulfate and chondroitin-6-sulfate), hyaluronic acid, heparin, heparin sulfate, heparan sulfate, alginic acid, pectin, carrageenan, dermatan sulfate, and dermatan-6-sulfate. These first polyanionic polysaccharides can be used singly or in a combination of two or more.

Examples of the water-soluble salt of the first polyanionic polysaccharide include inorganic salts, ammonium salts, and organic amine salts. Specific examples of the inorganic salts include: alkali metal salts such as sodium salts and potassium salts; alkali earth metal salts such as calcium salts; and metal salts such as zinc salts and iron salts.

The treatment liquid which is used for water-insolubilizing the film precursor section contains a first acid anhydride. Specific examples of the acid anhydride include acetic anhydride, propionic anhydride, succinic anhydride, butyric anhydride, phthalic anhydride, and maleic anhydride. Among them, acetic anhydride and propionic anhydride are preferable. These acid anhydrides can be used singly or in a combination of two or more.

It is preferable that the treatment liquid further contain at least one medium of water and a water-soluble organic solvent, and it is also preferable that the first acid anhydride be dissolved or dispersed in the medium. By using the treatment liquid in which the first acid anhydride is dissolved or dispersed in the medium, the film precursor section can be water-insolubilized sufficiently and immediately to form the film section.

Specific examples of the water-soluble organic solvent include methanol, ethanol, propanol, dimethyl sulfoxide (DMSO), acetonitrile, and tetrahydrofuran. Among them, methanol, ethanol, and dimethyl sulfoxide are preferable. These water-soluble organic solvents can be used singly or in a combination of two or more.

The concentration of the first acid anhydride in the treatment liquid is usually 0.1 to 50% by mass and is preferably 5 to 30% by mass. When the concentration of the first acid anhydride is less than 0.1% by mass, the extent of water-insolubilization for the film section to be formed is insufficient, or there is a tendency that the water-insolubilization takes a long time. On the other hand, when the concentration of the first acid anhydride exceeds 50% by mass, there is a tendency that the effects hit the ceiling.

The first polyanionic polysaccharide has a high hydrophilicity, and therefore it is preferable that the treatment liquid contain water as a medium from the viewpoint of water-insolubilizing the film precursor section further sufficiently and immediately. It is preferable to set the content of water in the treatment liquid to such an extent that the film precursor section cannot dissolve or swell. Specifically, the content of water in the treatment liquid is preferably 0.01 to 50% by mass, more preferably 5 to 20% by mass. When the content of water in the treatment liquid is less than 0.01% by mass, the water-insolubilization may be insufficient for the solvents other than methanol. In addition, when the content of water in the treatment liquid exceeds 50% by mass, keeping the shape of the film section to be formed may become difficult.

The reinforcement material which constitutes the medical material according to the present invention is (i) nonwoven fabric or woven fabric containing a biodegradable/absorbent polymer material or (ii) a sponge-like carrier or a felt-like carrier formed by water-insolubilizing a raw material-shaped body made of a raw material containing a second polyanionic polysaccharide with a treatment liquid containing a second acid anhydride.

The medical material according to the present invention is provided with the reinforcement material and the film section, and the film section penetrates at least a portion of the reinforcement material to have a structure integrated with the reinforcement material, so that the medical material according to the present invention has a moderate strength with which a suture and the like can be conducted. For example, the threading tension for the medical material according to the present invention is preferably 0.1 to 20 N/cm, more preferably 10 to 20 N/cm. When the threading tension is too low, there is a possibility that the reinforcement material is damaged after fixing the medical material. The threading tension does not need to be outstandingly higher than that of a suture thread for use in fixing the medical material.

In addition, the mass per unit area of the film section is preferably 5 to 100 g/m², more preferably 20 to 50 g/m². By setting the mass per unit area of the film section which constitutes the medical material within the range, the balance between the effect (anti-adhesion effect) when the medical material is used as the anti-adhesion material and the strength with which the suture and the like can be conducted becomes satisfactory.

As the biodegradable/absorbent polymer material which constitutes the nonwoven fabric and the woven fabric, it is preferable to use at least one selected from the group consisting of polylactic acid, lactic acid-caprolactone copolymers, and polyglycolic acid. Generally, an effect of suppressing or preventing adhesion cannot be obtained by placing the nonwoven fabric or the like containing a polymer material such as polylactic acid at an affected area of a living body. However, the medical material according to the present invention constituted by combining: the reinforcement material such as the nonwoven fabric containing a polymer material such as polylactic acid; and the film section formed using the first polyanionic polysaccharide exhibits the properties which are inherent in the first polyanionic polysaccharide, so that an effective anti-adhesion effect can be obtained.

The sponge-like carrier and the felt-like carrier are formed by insolubilizing the raw material-shaped body made of the raw material containing the water-soluble salt of the second polyanionic polysaccharide with the treatment liquid containing the second acid anhydride. As the second polyanionic polysaccharide, the same polyanionic polysaccharides as the first polyanionic polysaccharides can be used. The first polyanionic polysaccharide and the second polyanionic polysaccharide may be the same or different. In addition, as the second acid anhydride, the same acid anhydrides with the first acid anhydrides can be used. The first acid anhydride and the second acid anhydride may be the same or different.

To produce the sponge-like carrier, a sponge-like raw material-shaped body is formed, for example, by pouring an aqueous solution of the water-soluble salt of the second polyanionic polysaccharide into an appropriate container and then drying or freeze-drying the aqueous solution. Subsequently, the sponge-like raw material-shaped body is water-insolubilized with the treatment liquid containing the second acid anhydride to obtain the sponge-like carrier. In addition, the felt-like carrier can be obtained, for example, by pressing the sponge-like carrier obtained in the manner as described above, or other methods.

To produce the medical material according to the present invention, the reinforcement material is first immersed in the raw material containing the water-soluble salt of the first polyanionic polysaccharide to impregnate at least a portion of the reinforcement material with the raw material. Subsequently, the reinforcement material is subjected to drying or the like, thereby obtaining a composite film provided with the reinforcement material and the film precursor section penetrating at least a portion of the reinforcement material to be integrated with the reinforcement material. Thereafter, the film precursor section is water-insolubilized using the treatment liquid containing the first acid anhydride to form a film section, and thereby, the medical material according to the present invention can be obtained. It is to be noted that the raw material containing the water-soluble salt of the first polyanionic polysaccharide may further contain a radiopaque agent including a contrast agent such as barium sulfate.

By treating the composite film with the treatment liquid containing the first acid anhydride, the film precursor section is water-insolubilized while the composite film maintains the shape thereof. The method for treating the composite film with the treatment liquid is not particularly limited; however, the treatment is preferably conducted in such a way that the treatment liquid is brought into contact with the whole composite film and the treatment liquid penetrates inside the film precursor section. Specific examples of the treatment method include a method in which the composite film is immersed in the treatment liquid and a method in which the treatment liquid is applied or sprayed (atomized) onto the composite film.

The temperature during the water-insolubilization treatment is not particularly limited as long as the temperature does not exceed the boiling point of the treatment liquid. It is preferable to set the temperature during the water-insolubilization treatment at 0 to 80°C, more preferably 0 to 70°C, and particularly preferably room temperature (25°C) to 60°C from the viewpoint of suppressing the decomposition and denaturation of the polyanionic polysaccharide and from the viewpoint of suppressing the volatilization of the medium, byproducts, and the like. However, when the treatment is conducted under the condition in which the treatment liquid does not volatilize during the water-insolubilization treatment, for example, the treatment is conducted with a heat press or a heat roller, the medical material can be obtained in a shorter time without undergoing the decomposition and denaturation, and the like. For example, in the case where the water-insolubilization treatment is conducted with a heat press or a heat roller, it is preferable that the temperature during the water-insolubilization treatment be set at 50 to 90°C, and the treatment time be set for 30 minutes or shorter. After the water-insolubilization treatment is completed, the medical material according to the present invention can be obtained, if necessary, through washing or the like with water or a water-soluble organic solvent.

The reaction that is supposed to progress when the film precursor section formed using a sodium salt of the polyanionic polysaccharide is treated with an alcoholic solution of acetic anhydride is shown below. It is to be noted that the supposed reaction can be one of the factors for water-insolubilization, but there is a possibility that the water-insolubilization is achieved by a combination with another factor for water-insolubilization or by a totally different factor. That is, the present invention is not limited at all by the supposed reaction described below.

R₁-COON^{a} + (CH₃CO)₂O + R₂-OH → R₁-COOH + CH₃COONa + CH₃COOR₂ ^{...} (1)

In the reaction formula (1), R₁ represents the main chain of the polyanionic polysaccharide, and R₂ represents the main chain of the alcohol. Acetic anhydride, when cleaved in the presence of an alcohol, deprives sodium of the polyanionic polysaccharide and the carboxy groups change into an acid form from a sodium salt form. The change can be confirmed by measuring the Na content or by titration with an alkaline solution.

In the case where water exists in the reaction system, it is envisaged that the reaction represented by the following reaction formula (2) progresses in addition to and in parallel with the reaction represented by the reaction formula (1) and the carboxy groups change into an acid form from a sodium salt form.

R₁-COON^{a} + (CH₃CO)₂O + H₂O → R₁-COOH + CH₃COON^{a} + CH₃COOH ^{...} (2)

It is to be noted that all the anionic groups in a molecule may not necessarily be in the acid form in the film section of the obtained medical material.

It is extremely difficult to obtain a shaped body that is sufficiently water-insolubilized even when a shaped body or the like formed using the water-soluble salt of the polyanionic polysaccharide is immersed in an inorganic acid such as hydrochloric acid or an organic acid such as acetic acid. In addition, a water-insolubilized, shaped body cannot be obtained even when the acid anhydride in the treatment liquid is replaced with an acid that corresponds to the acid anhydride. From these facts, it is envisaged that the water-insolubilization is achieved by a different factor combined with the factor that the anionic groups in the polyanionic polysaccharide change into the acid form.

There is no need to use a chemical crosslinking agent during the production of the medical material according to the present invention, and therefore a structure of a functional group or the like which is derived from the chemical crosslinking agent is not incorporated into the molecule. Therefore, the medical material according to the present invention retains the properties which are inherent in the polyanionic polysaccharide being a raw material and has a high level of safety. Accordingly, the medical material according to the present invention is suitable as an anti-adhesion material and the like. It is to be noted that in the case where the medical material according to the present invention is used as a constituent material for an anti-adhesion material, the thickness of the medical material is not particularly limited, but is preferably 20 to 200 µm, more preferably 60 to 120 µm.

The molecules of the polyanionic polysaccharide which constitutes the medical material according to the present invention are not substantially crosslinked. Further, a new covalent bond is not substantially formed in the polyanionic polysaccharide. However, it is inferred that physical bonds such as hydrogen bonds, hydrophilic bonds, and van der Walls force are formed between the molecules of the polyanionic polysaccharide. Formation of such physical bonds between the molecules of the polyanionic polysaccharide can be confirmed by measuring an infrared absorption spectrum.

The film section which constitutes the medical material according to the present invention is stably water-insoluble in a wide pH range from acidity to alkalinity. However, the film section which constitutes the medical material according to the present invention, when brought into contact with or immersed in an aqueous medium having a pH of 12 or higher, can be dissolved easily due to dissociation of the physical bonds between the molecules.

### (Anti-Adhesion Material)

The anti-adhesion material according to the present invention contains a polyhydric alcohol or a polyhydric alcohol aqueous solution retained in the medical material. Specific examples of the polyhydric alcohol include ethylene glycol, diethylene glycol, polyethylene glycol, methyl glycerol, polyoxyethylene glycoside, maltitol, mannitol, xylitol, sorbitol, reduced sugar syrup, dipropylene glycol, butylene glycol, valine, propylene glycol, glycerin (glycerol), polyglycerin, and fatty acid esters of glycerin. Among them, polyhydric alcohols which are used in the medical field and the food field, such as glycerin, xylitol, sorbitol, and low-molecular-weight polyethylene glycols, are used suitably. With respect to these suitably usable polyhydric alcohols, products sold on the market can be used as they are. With respect to glycerin, sorbitol, and the like, it is desirable to use products which conform to the Japanese pharmacopoeia. Glycerin is a material having a high level of safety to such an extent that can also be used as injections into a vein and therefore is greatly preferable.

Examples of the method for allowing the polyhydric alcohol or the polyhydric alcohol aqueous solution to be retained in the medical material include a method in which the medical material is immersed in the polyhydric alcohol or the polyhydric alcohol aqueous solution having a predetermined concentration. That is, by immersing the medical material in the polyhydric alcohol aqueous solution to replace the inside of the film section with the polyhydric alcohol aqueous solution, the polyhydric alcohol aqueous solution in a desired concentration is retained, so that a desired anti-adhesion material according to the present invention can be obtained. It is to be noted that the thickness of the anti-adhesion material according to the present invention is not particularly limited, but is preferably 20 to 2000 µm, more preferably 60 to 1200 µm.

### Examples

Hereinafter, the present invention will be described specifically based on Examples; however, the present invention is not limited to the Examples. It is to be noted that the "part (s) " and "%" in Examples and Comparative Examples are on a mass basis unless otherwise noted.

### (Example 1)

Woven fabric of polylactic acid derived from a plant raw material (mesh count of 97 threads/inch, opening ratio of 62%, thickness of 90 µm) was cut in a size of 12-cm length × 10-cm width and was laid on a stainless-steel tray. Into the tray, 30 mL of a 1% sodium hyaluronate (molecular weight of 800000 Da) aqueous solution were poured to impregnate the woven fabric of polylactic acid, and then the woven fabric was dried in a thermostatic chamber at 20°C to obtain a woven fabric of polylactic acid-sodium hyaluronate composite film. The obtained composite film was immersed in a treatment liquid (solution of 20% acetic anhydride/80% ethanol) and was then left to stand at 50°C for 1 hour to be water-insolubilized and thereby obtain a woven fabric of polylactic acid-hyaluronic acid composite film (medical material) having a thickness of about 100 µm. With respect to the obtained woven fabric of polylactic acid-hyaluronic acid composite film, the mass per unit area of the film section (hyaluronic acid) was 40 g/m², and the threading tension was 12.1 N/cm.

### (Example 2)

A nonwoven fabric of polyglycolic acid-hyaluronic acid composite film (medical material) having a thickness of about 1000 µm was obtained in the same manner as in Example 1 except that nonwoven fabric of polyglycolic acid (product name "Biofelt", manufactured by COREFRONT Corporation, thickness of 1 mm) was used in place of the woven fabric of polylactic acid. With respect to the obtained nonwoven fabric of polyglycolic acid-hyaluronic acid composite film, the mass per unit area of the film section (hyaluronic acid) was 70 g/m², and the threading tension was 19.5 N/cm.

### (Example 3)

Into a stainless-steel tray of 12-cm length × 10-cm width, 50 mL of a 1% sodium hyaluronate (molecular weight of 800000 Da) aqueous solution were poured and then frozen in a freezer at -80°C. The frozen aqueous solution was subjected to vacuum freeze drying (degree of vacuum of -20 Pa, shelf temperature of 25°C) to obtain a sponge-like carrier made of sodium hyaluronate. The obtained sponge-like carrier was immersed in a treatment liquid (solution of 20% acetic anhydride/80% ethanol) and was then left to stand at 50°C for 1 hour to be water-insolubilized and thereby obtain a sponge-like carrier made of hyaluronic acid. The obtained sponge-like carrier was impregnated with 50 mL of a 1% sodium hyaluronate (molecular weight of 800000 Da) aqueous solution and was then dried in a thermostatic chamber at 20°C to obtain a sponge-like carrier-sodium hyaluronate composite film. The obtained composite film was immersed in the treatment liquid (solution of 20% acetic anhydride/80% ethanol) and was then left to stand at 50°C for 1 hour to be water-insolubilized and thereby obtain a sponge-like carrier-hyaluronic acid composite film (medical material) having a thickness of about 120 µm. With respect to the obtained sponge-like carrier-hyaluronic acid composite film, the mass per unit area of the film section (hyaluronic acid) was 50 g/m², and the threading tension was 10.5 N/cm.

### (Example 4)

The sponge-like carrier made of hyaluronic acid and obtained in Example 3 was pressed at about 10 kgf using a hand press to obtain a felt-like carrier. Subsequently, a felt-like carrier-hyaluronic acid composite film (medical material) having a thickness of about 90 µm was obtained in the same manner as in Example 3 except that the felt-like carrier obtained above was used in place of the sponge-like carrier made of hyaluronic acid. With respect to the obtained felt-like carrier-hyaluronic acid composite film, the mass per unit area of the film section (hyaluronic acid) was 50 g/m², and the threading tension was 13.4 N/cm.

### (Evaluation 1: Solubility Test)

The composite films produced in respective Examples were each cut into a 2-cm square and put into a container having a diameter of 3.5 cm and a depth of 1.5 cm, and 5 mL of a PBS buffer solution (pH of 6.8) were added thereto. The container was placed in a shaker the temperature of which was adjusted at 37°C and was then shaken at 10 to 20 rpm, and the changes of the state over time were visually observed. As a result, it was understood that the original shape of the film was retained even after 72 hours and the film was water-insolubilized for any of the composite films. In addition, the swelling ratio (swollen film/dried film (mass ratio)) after 72 hours was 2.3.

### (Example 5)

The composite film produced in Example 1 was immersed in a 10% by volume glycerin aqueous solution. The composite film was then air-dried and thereafter sealed in a sterilization bag. Sterilization was conducted to the composite film together with the sterilization bag through irradiation with 25 kGy radiation rays to obtain an anti-adhesion film having a thickness of about 100 µm. A laparotomy was performed to a mature dog (beagle dog, female, 1.5 years old, weight of about 10 kg) after general anesthesia, and the epidermis of a ventral wall was peeled into a 3-cm square. The anti-adhesion film was placed so as to cover the peeled portion, and an abdominal closure was then performed. Two weeks later, a laparotomy was performed to the same dog after general anesthesia to find that adhesion had not occurred. In addition, the anti-adhesion film placed (implanted) in the body of the dog was found to have disappeared two weeks after the implantation. As the reason, it is inferred that the carboxy groups in hyaluronic acid, which constitutes the anti-adhesion film, were gradually neutralized by sodium ions or the like in the living body to cause the hyaluronic acid to change into a water-soluble hyaluronic acid salt, so that the hyaluronic acid dissolved to be absorbed into the living body. In contrast, with respect to a dog to whom an abdominal closure was performed without placing the anti-adhesion film, it was observed that adhesion had occurred between the peeled portion and the bowel. In addition, with respect to a dog to whom an abdominal closure was performed placing only woven fabric of polylactic acid in place of the anti-adhesion film, it was observed that adhesion had occurred between the peeled portion and the bowel.

### Industrial Applicability

The medical material according to the present invention is useful as a material for constituting an anti-adhesion material.

## Claims

1. A medical material comprising:
a reinforcement material; and
a film section penetrating at least a portion of the reinforcement material to be integrated with the reinforcement material, wherein:
the film section is formed by water-insolubilizing a film precursor section made of a raw material comprising a water-soluble salt of a first polyanionic polysaccharide with a treatment liquid comprising a first acid anhydride; and
the reinforcement material is: nonwoven fabric or woven fabric comprising a biodegradable/absorbent polymer material; or
a sponge-like carrier or a felt-like carrier formed by water-insolubilizing a raw material-shaped body made of a raw material comprising a water-soluble salt of a second polyanionic polysaccharide with a treatment liquid comprising a second acid anhydride.

2. The medical material according to claim 1, wherein a mass per unit area of the film section is 5 to 100 g/m².

3. The medical material according to claim 1 or 2, having a threading tension of 0.1 to 20 N/cm.

4. The medical material according to any one of claims 1 to 3, wherein the first polyanionic polysaccharide and the second polyanionic polysaccharide are each at least one selected from the group consisting of hyaluronic acid, carboxymethyl cellulose, and alginic acid.

5. The medical material according to any one of claims 1 to 4, wherein the first acid anhydride and the second acid anhydride are each at least any one of acetic anhydride and propionic anhydride.

6. The medical material according to any one of claims 1 to 5, wherein the polymer material is at least one selected from the group consisting of polylactic acid, lactic acid-caprolactone copolymers, and polyglycolic acid.

7. An anti-adhesion material comprising a polyhydric alcohol or a polyhydric alcohol aqueous solution retained in the medical material according to any one of claims 1 to 6.
